# EUROPÄISCHE PATENTSCHRIFT

(11) **EP 0 695 161 B1**
(45) Veröffentlichungstag und Bekanntmachung des Hinweises auf die Patenterteilung: **01.04.1998**
(21) Anmeldenummer: 94913592.5
(22) Anmeldetag: 13.04.1994
(51) Int. Cl.: A61K 7/00, A61K 7/48

(54) **KOSMETISCHE UND MEDIZINISCHE TOPISCHE ZUBEREITUNGEN**
COSMETIC AND MEDICINAL TOPICAL PREPARATIONS
PREPARATIONS TOPIQUES COSMETIQUES ET MEDICALES

(30) Priorität: 19.04.1993 DE 4312656
(43) Veröffentlichungstag der Anmeldung: 07.02.1996
(73) Patentinhaber: Beiersdorf Aktiengesellschaft, 20245 Hamburg (DE)
(72) Erfinder: GOHLA, Sven, D-22607 Hamburg (DE); HEINZE, Friedrich, D-60317 Frankfurt am Main (DE); NIELSEN, Jens, D-22844 Norderstedt (DE); THAMSSEN, Carl, D-22459 Hamburg (DE)
(86) Internationale Anmeldenummer: EP9401145
(87) Internationale Veröffentlichungsnummer: WO9423688

(56) Entgegenhaltungen:
- EP-A- 0 259 982
- EP-A- 0 303 461
- DE-A- 3 039 468
- PATENT ABSTRACTS OF JAPAN vol. 9, no. 205 (C-299) & JP,A,60 075 405 (SHISEIDO KK) 27. April 1985 & DATABASE WPI Week 8523, Derwent Publications Ltd., London, GB; AN 85-138963 & JP,A,60 075 405 (SHISEIDO KK) 27. April 1985
- DATABASE WPI Week 8906, Derwent Publications Ltd., London, GB; AN 89-042223 & JP,A,63 313 709 (LION CORP.) 21. Dezember 1988
- DATABASE WPI Week 9119, Derwent Publications Ltd., London, GB; AN 91-136885 & JP,A,03 174 316 (KANEBO KK) 28. März 1991
- DATABASE WPI Week 8530, Derwent Publications Ltd., London, GB; AN 85-181162 & JP,A,60 109 514 (SHISEIDO KK) 15. Juni 1985
- DATABASE WPI Week 8223, Derwent Publications Ltd., London, GB; AN 82-47087E & JP,A,57 070 824 (NIPPON SURFACTANT KK) 1. Mai 1982
- DATABASE WPI Week 8537, Derwent Publications Ltd., London, GB; AN 85-226610 & JP,A,60 146 812 (YG NONOKAWA SHOJI) 2. August 1985

## Beschreibung

Die vorliegende Erfindung betrifft pflegende kosmetische und medizinische topische Zubereitungen, insbesondere solche, die, auf der Haut oder Schleimhäuten angewandt, befeuchtend und kühlend wirken.

Solche Zusammensetzungen sind an sich bekannt. In der Literatur werden beispielsweise ionische Verbindungen, insbesondere Ammoniumsalze, als kühlende Agenzien beschrieben. Als kühlende Zubereitungen werden auch verbreitet isopropanolische Gele mit Campher- und Mentholzusatz angewandt.

Allgemein werden häufig etherische Öle, vornehmlich Campher und Menthol, aber auch deren Derivate, z.B. Menthyllactat oder Menthyl-3-hydroxybutyrat, in kühlende Zusammensetzungen eingearbeitet.

Menthol und Campher und deren Derivate, aber auch andere etherische Öle erniedrigen die Reizschwelle der neuronalen Kälterezeptoren und rufen so ein Kältegefühl hervor. Häufig bewirken sie aber gleichzeitig eine Durchblutungssteigerung, die im Gegenteil ein Wärmegefühl hervorruft.

Die Anwendung dieser Substanzen, namentlich auf gereizter Haut, ist jedenfalls problematisch. Darüber hinaus sind viele dieser Verbindungen schlecht wasserlöslich. Ihre Verwendung ist folglich auf wenige Kosmetika und Dermatika beschränkt.

Aufgabe der vorliegenden Erfindung war es also, pflegende kosmetische und medizinische Zubereitungen zur Verfügung zu stellen, die nicht die Nachteile des Standes der Technik haben, insbesondere solche, welche, auf der Haut oder Schleimhäuten angewandt, befeuchtend und/oder kühlend wirken.

Erfindungsgemäß wird diese Aufgabe gelöst durch die Verwendung kosmetisch oder pharmazeutisch unbedenklicher Substanzen mit positiver Lösungsenthalpie zur Herstellung kosmetischer oder medizinisch topischer Zubereitungen zur Pflege der Haut, wobei die Substanz oder die Substanzen in den Zubereitungen in einem weitgehend wasserfreien Medium vorliegen und/oder durch eine stoffliche Barriere von einem wasserhaltigen Medium abgeschirmt werden.

Als besonders vorteilhafte Verkörperung der vorliegenden Erfindung wird die Aufgabe gelöst durch kosmetische und dermatologische Zusammensetzungen, enthaltend einen oder mehrere Zuckeralkohole der allgemeinen Formel 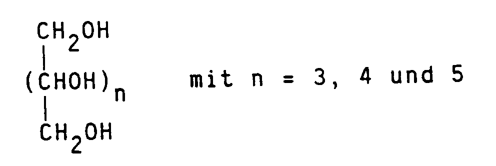 wobei der oder die Zuckeralkohole in den Zubereitungen in einem weitgehend wasserfreien Medium vorliegen und/oder durch eine stoffliche Barriere von einem wasserhaltigen Medium abgeschirmt werden.

Es ist bekannt, daß einige Zuckeralkohole beim Auflösen in Wasser unter Normalbedingungen positive Lösungsenthalpie haben, d.h., daß sich die Lösung wahrend des Lösungsvorganges abkühlt. Die höchste positive Lösungsenthalpie unter den Zuckeralkoholen hat Xylitol. Xylitol ist der bevorzugte, erfindungsgemäß verwendete Zuckeralkohol.

Xylitol hat in der Fischer-Schreibweise die Strukturformel

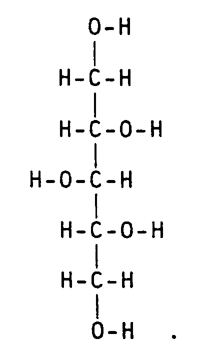

Es hat meso-Konfiguration und ist infolgedessen nicht optisch aktiv.

Xylitol (xylo-Pentanpentol) kann technisch durch Hydrierung von Xylose gewonnen werden und wird z.B. als Zuckerersatzstoff für Diabetiker eingesetzt. Es dient gelegentlich in kosmetischen Zubereitungen als Feuchthaltemittel.

Unbekannt war indes, die positive Lösungsenthalpie von kosmetisch oder pharmazeutisch unbedenklichen Stoffen im allgemeinen oder der erfindungsgemäßen Zuckeralkohole im besonderen für die Verwendung in kühlend wirkenden kosmetischen oder medizinischen topischen Zubereitungen zu nutzen.

Weitere erfindungsgemäß wirksame Substanzen sind Uronsäuren der allgemeinen Formel 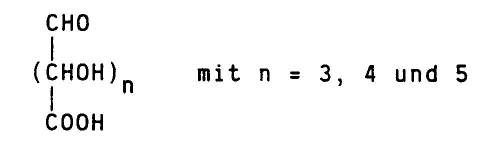 (bzw. deren cyclische Formen) und deren Salze, sofern sich diese Uronsäuren bzw. Uronsäuresalze durch positive Lösungsenthalpie und durch kosmetische bzw. pharmazeutische Unbedenklichkeit auszeichnen.

Ein erfindungsgemäß vorteilhaftes Uronsäuresalz ist Natriummannuronat, welches sich durch die folgende Struktur auszeichnet:

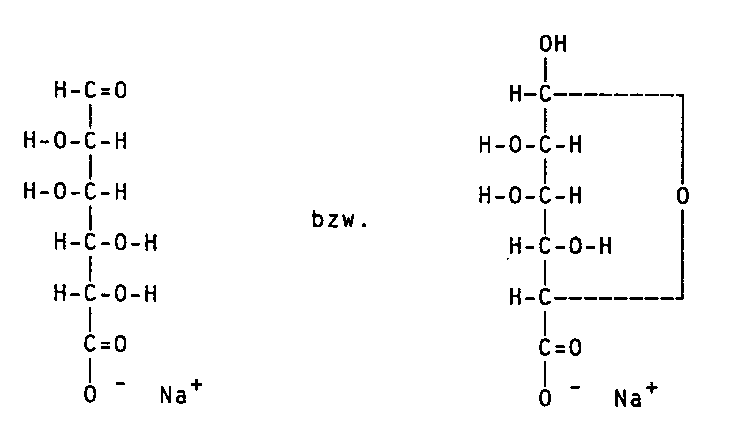

Besonders überraschend war, daß sich die kühlende Wirkung von Xylitol erfindungsgemäß in Kombinationen mit Stoffen, gewählt aus der Gruppe Mannitol, Sorbitol, Propylenglycol, Glycerin, Harnstoff, 2-Pyrrolidon-5-carbonsäure und deren Natriumsalz sowie Natriummannuronat noch steigern läßt. Die Steigerung der Kühlwirkung ist in Bezug auf die Kühlwirkung der Einzelsubstanzen überadditiv und beruht somit auf einem Synergismus.

Die Mischungen aus Xylitol und dieser Stoffgruppe zeichnen sich zudem überraschenderweise durch besonders günstige hautpflegende Eigenschaften aus. Insbesondere wird die Fähigkeit des Xylitols, der Haut Feuchtigkeit zuspenden, deutlich gesteigert.

Es ist im Sinne der vorliegenden Erfindung möglich und gegebenenfalls vorteilhaft, solche Mischungen auch in stark wasserhaltigen kosmetischen oder medizinischen topischen Zubereitungen einzusetzen. Dabei tritt die kühlende Wirkung gegebenenfalls etwas in den Hintergrund.

Vorteilhaft sind insbesondere Mischungen aus Xylitol und mindestens einem der Stoffe, gewählt aus der Gruppe Mannitol, Sorbitol, Glycerin, Propylenglycol, Harnstoff, 2-Pyrrolidon-5-carbonsäure, Natrium-(2-Pyrrolidon-5-)carboxylat, Natriummannuronat, NH₄Cl und Weinsäure und/oder deren Salze.

In solchen Mischungen kann der vorteilhaft Xylitolgehalt 0,5 - 99,5 Gew.-% betragen, bezogen auf das Gesamtgewicht dieser Zusammensetzungen.

Vorteilhaft sind ferner Zusammensetzungen, enthaltend
0,5 - 10,0 Teile Xylitol
0,0 - 5,0 Teile Mannitol
0,0 - 5,0 Teile Sorbitol
0,0 - 5,0 Teile Glycerin
0,0 - 5,0 Teile Propylenglycol
0,0 - 5,0 Teile Harnstoff
0,0 - 5,0 Teile 2-Pyrrolidon-5-carbonsäure
0,0 - 5,0 Teile Natrium-(2-Pyrrolidon-5-)carboxylat
0,0 - 5,0 Teile Natriummannuronat,
0,0 - 5,0 Teile NH₄Cl
0,0 - 5,0 Teile eines oder mehrerer Weinsäuresalze
bezogen auf die Gesamtzusammensetzung der Zubereitung, mit der Maßgabe, daß mindestens einer der Nicht-Xylitol-Bestandteile in einer Konzentration von wenigstens 0,1 Gew.-% vorliegt.

Bevorzugt enthalten diese Kombinationen
1,0 - 8,0 Teile Xylitol
0,0 - 3,0 Teile Mannitol
0,0 - 3,0 Teile Sorbitol
0,0 - 3,0 Teile Glycerin
0,0 - 3,0 Teile Propylenglycol
0,0 - 3,0 Teile Harnstoff
0,0 - 3,0 Teile 2-Pyrrolidon-5-carbonsäure
0,0 - 3,0 Teile Natrium-(2-Pyrrolidon-5-)carboxylat
0,0 - 3,0 Teile Natriummannuronat ,
0,0 - 3,0 Teile NH₄Cl
0,0 - 3,0 Teile eines oder mehrerer Weinsäuresalze
bezogen auf die Gesamtzusammensetzung der Zubereitung, mit der Maßgabe, daß mindestens einer der Nicht-Xylitol-Bestandteile in einer Konzentration von wenigstens 0,5 Gew.-% vorliegt.

Ganz besonders bevorzugt enthalten diese Kombinationen
2,0 - 5,0 Teile Xylitol
0,0 - 2,5 Teile Mannitol
0,0 - 2,5 Teile Sorbitol
0,0 - 2,5 Teile Glycerin
0,0 - 2,5 Teile Propylenglycol
0,0 - 2,5 Teile Harnstoff
0,0 - 2,5 Teile 2-Pyrrolidon-5-carbonsäure
0,0 - 2,5 Teile Natrium-(2-Pyrrolidon-5-)carboxylat
0,0 - 2,5 Teile Natriummannuronat ,
0,0 - 2,5 Teile NH₄Cl
0,0 - 2,5 Teile eines oder mehrerer Weinsäuresalze
bezogen auf die Gesamtzusammensetzung der Zubereitung, mit der Maßgabe, daß mindestens einer der Nicht-Xylitol-Bestandteile in einer Konzentration von wenigstens 1,0 Gew.-% vorliegt.

Die erfindungsgemäße stoffliche Barriere kann beispielsweise darin bestehen, die Substanz oder die Substanzen mit positiver Lösungsenthalpie mikroverkapselt einzusetzen.

Wird gewünscht, daß die Substanz oder die Substanzen in den Zubereitungen durch Mikroverkapselung von einem wasserhaltigen Medium abgeschirmt werden, so können die für diese Methode üblichen Verfahren eingesetzt werden. Typisch ist, das Hüllmaterial in einem Lösemittel - kolloidal oder als echte Lösung - zu lösen und das Kernmaterial, also den späteren Inhalt der Mikrokapseln, in der so entstandenen Lösung als Festkörper oder als feinste Tröpfchen zu dispergieren.

Die besagte Dispersion wird, in feinste Tröpfchen zerteilt, in ein erhitztes Medium, z.B. heiße Luft, versprüht. Dabei verdunstet das Lösemittel. Das Hüllmaterial fällt wieder als Festkörper aus und bildet eine Hülle um das Kernmaterial. Damit liegen bereits rohe Mikrokapseln vor, die noch den üblichen Aufbereitungsschritten unterworfen und den endgültigen Zubereitungen einverleibt werden können. Dieses Verfahren nutzt das bekannte Phänomen der Koazervation.

Eine andere Möglichkeit besteht darin, die Hülle der Mikrokapseln durch Grenzflächenpolymerisation des Hüllmaterials zu erzeugen. Dies bedeutet, daß nicht das endgültige Hüllmaterial als solches eingesetzt wird, sondern Vorstufen, beispielsweise Monomere, die sich auf dem Kernmaterial anlagern und dort zur endgültigen Hüllfolie polymerisieren.

Fettcoating-Verfahren stellen ebenfalls vorteilhafte Verkörperungen der erfindungsgemäßen Verfahren dar.

Die Materialien für die Mikroverkapselung können vorteilhaft aus den üblichen hydrophilen oder hydrophoben Stoffen oder Mischungen daraus gewählt werden. Feststoffe, insbesondere natürliche polymere Materialien, z.B. Stärke und andere Polysaccharide, sind bevorzugt. Aber auch synthetische Polymere können vorteilhaft verwendet werden.

Beispiele für Hüllmaterialien sind Fette und/oder Wachse, vorzugsweise solche mit einer Erstarrungstemperatur von ca. 35 - 80° C. Besonders vorteilhaft sind Abmischungen aus Cetylpalmitat und Cetylalkohol.

Ferner sind vorteilhaft: Polysaccharide und deren Derivate natürlicher sowie partialsynthetischer Herkunft, insbesondere Cellulosederivate, worunter im besonderen auch die Derivate des Chitins zu verstehen sind. Ferner die Polymerisate der α- und/oder β-Hydroxycarbonsäuren, insbesondere die Polymerisate der Glycolsäure (Polyglycolide), der Milchsäure (Polylactide), der α-Hydroxybuttersäure (Polyhydroxybutyrate), der α-Hydroxyvaleriansäure (Polyhydroxyvalerate) und/oder deren Copolymerisate bzw. Mischungen aus solchen Polymerisaten und/oder Copolymerisaten.

In jedem Falle, unabhängig vom gewählten Herstellungsverfahren der erfindungsgemäßen Mikrokapseln, ist bevorzugt, bei einer Temperatur zu arbeiten, welche den Schmelzpunkt des Kernmaterials nicht überschreitet. Im Falle reinen Xylitols bedeutet dies, daß die Arbeitstemperatur nicht mehr als 90° C betragen sollte.

Die Mikrokapseln können, wie dem Fachmann bekannt ist, auf mancherlei Weise geöffnet werden. So können sie durch mechanische Krafteinwirkung aufgebrochen oder auch durch chemische, z.B. enzymatische, Vorgänge geöffnet werden und den oder die Wirkstoffe aus dem Kern der Kapsel freigeben.

Es ist ferner günstig, wenn das Kernmaterial im Lösemittel möglichst wenig löslich ist.

Die erfindungsgemäßen kosmetischen und/oder dermatologischen Zubereitungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden. Sie enthalten bevorzugt 0,01 Gew.-% bis 30 Gew.-%, insbesondere aber 0,1 Gew.-% bis 10 Gew.-%, an kosmetisch oder pharmazeutisch unbedenklichen Substanzen mit positiver Lösungsenthalpie, insbesondere Zuckeralkohole, besonders bevorzugt Xylitol, sofern gewährleistet ist, daß die Substanz oder die Substanzen in den Zubereitungen in einem weitgehend wasserfreien Medium vorliegen und/oder durch eine stoffliche Barriere von einem wasserhaltigen Medium abgeschirmt werden.

Zur Anwendung werden die erfindungsgemäßen Zubereitungen in der für Kosmetika und Dermatika üblichen Weise in ausreichender Menge auf die Haut aufgebracht.

Die erfindungsgemäßen Zubereitungen können in verschiedenen Formen vorliegen. So können sie z.B. eine Lösung, eine Emulsion vom Typ Wasser-in-Öl (W/O) oder vom Typ Öl-in-Wasser (O/W), oder Mehrfachemulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Si likonderivate.

Sofern die kosmetische oder dermatologische Zubereitung eine Lösung oder Emulsion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Pro pylenglykol, Glycerin, Ethylenglykol, Ethylenglykolmonoethyl- oder -monobutylether, Propylenglykolmonomethyl-, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist. Ferner können solche Gele Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Ver dickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Feste Stifte gemäß der Erfindung enthalten z.B. natürliche oder synthetische Wachse, Fettalkohole, Metallseifen oder Fettsäureester.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die erfindungsgemäßen Zubereitungen können vorteilhaft in Form von desodorierenden Kosmetika vorliegen. In diesem Falle enthalten sie einen oder mehreren Stoff aus der Gruppe der Substanzen, welche üblicherweise als desodorierend oder antitranspirierend wirkende Stoffe Verwendung finden. Vorteilhaft sind Aluchlorhydrat, aber auch Gemische wie sie beispielsweise in der DE-OS 37 40 186 beschrieben werden.

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die UVB-Filter können öllöslich oder wasserlöslich sein. Vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter soll selbstverständlich nicht limitierend sein.

UVA-Filter, die erfindungsgemäß vorteilhaft verwendet werden können, sind beispielsweise Derivate des Dibenzoylmethans, insbesondere 1-(4'-tert.Butylphenyl)-3-(4'-methoxyphenyl)propan-1,3-dion und um 1-Phenyl-3-(4'-isopropylphenyl)propan-1,3-dion. Es können die für die UVB-Kombination verwendeten Mengen eingesetzt werden.

Die Liste der genannten UVA-Filter soll selbstverständlich ebenfalls nicht limitierend sein.

Die erfindungsgemäßen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Vorteilhaft sind auch solche kosmetische und dermatologische Zubereitungen, die in der Form eines Sonnenschutzmittels, eines Pré-Soleil- oder Après-Soleil-Produktes vorliegen. Vorteilhaft enthalten diese zusätzlich mindestens einen UVA-Filter und/oder mindestens einen UVB-Filter und/oder mindestens ein anorganisches Pigment.

Die nachfolgenden Beispiele dienen dazu, die vorliegende Erfindung näher zu erläutern, ohne daß aber beabsichtigt ist, die Erfindung auf diese Beispiele zu beschränken.

In den Beispielen 1 - 10 werden erfindungsgemäße vorteilhafte Kombinationen aufgeführt.

Diese Kombinationen oder die erfindungsgemäßen Einzelsubstanzen, vorteilhaft das Xylitol selbst, können, wie in Beispielen 11 - 13 gezeigt wird, mikroverkapselt werden, und, wie Beispiele 14 - 29 zeigen, in kosmetische oder medizinische topische Zubereitungen eingearbeitet werden.

| Beispiel | 1 | 2 | 3 | 4 | 5 |
|---|---|---|---|---|---|
| Xylitol | 5,00 | 10,00 | 3,00 | 10,00 | 7,50 |
| Mannitol | 1,00 | 1,00 | - | 2,00 | 1,00 |
| Sorbitol | 0,30 | - | 1,00 | - | 1,00 |
| Glycerin | 2,00 | - | - | 2,00 | 2,00 |
| PG | 1,00 | - | - | 1,00 | - |
| Harnstoff | 2,00 | 1,00 | - | 1,00 | 3,00 |
| PCA | 1,00 | - | 1,00 | - | - |
| NaPCA | 0,50 | - | - | - | - |
| NaMu | 0,30 | - | - | - | - |
| NH₄Cl | 2,00 | 3,00 | 3,00 | 1,50 | 1,00 |
| NaTa | 1,50 | 2,00 | 1,00 | 2,50 | 3,00 |

| Beispiel | 6 | 7 | 8 | 9 | 10 |
|---|---|---|---|---|---|
| Xylitol | 16,50 | 4,50 | 6,00 | 8,00 | 7,00 |
| Mannitol | 3,50 | 2,30 | 1,00 | - | 2,00 |
| Sorbitol | - | 1,00 | 1,00 | - | - |
| Glycerin | 2,00 | 2,00 | - | 1,00 | 3,00 |
| PG | - | 2,00 | - | - | - |
| Harnstoff | 3,00 | - | 1,00 | 1,00 | 1,00 |
| PCA | - | - | 1,00 | - | - |
| NaPCA | - | 1,00 | - | - | - |
| NaMu | 1,00 | - | - | - | - |
| NH₄Cl | 1,50 | 3,00 | 1,00 | 1,00 | 5,00 |
| NaTa | 1,50 | 2,00 | 2,50 | 3,00 | 1,00 |
| PG = Propylenglycol PCA = 2-Pyrrolidon-5-carbonsäure NaPCA = Natrium-(2-Pyrrolidon-5-)carboxylat NaMu = Natriummannuronat NaTa = Natriumtartrat ( = C₄H₄Na₂O₆ * 2 H₂O) | | | | | |

Die Zahlenwerte beziehen sich auf Gewichtsteile.

### Beispiel 11

Xylitol wird in mikronisierter Form vorgelegt. Über eine Düse wird ein Gemisch aus Polylactid und Methylcellulose im Verhältnis 1 : 10, welches in Chloroform gelöst vorliegt, und welches auf eine Temperatur von 60° C erwärmt wurde, im Wirbelschichtverfahren auf die Xylitolpartikel aufgebracht.

Es werden Mikrokapseln mit einem Gehalt an in fester Form vorliegenden Xylitol in hoher Ausbeute erhalten.

### Beispiel 12

Xylitol wird in mikronisierter Form in einen für die Mischung von Pulver geeigneten Knetmischer gegeben. Ein Triglyceridgemisch (Witepsol^{R}, hochschmelzende Varianten, Dynamit Nobel) wird bei 40° C mit dem mikronisierten Xylitol vereinigt. Das so entstandene Gemisch wird unter ständiger kräftiger Agitation auf Raumtemperatur abgekühlt.

Es werden Mikrokapseln mit einem Gehalt an in fester Form vorliegenden Xylitol in hoher Ausbeute erhalten.

### Beispiel 13

Ein Gemisch gemäß Beispiel 1 wird in mikronisierter Form vorgelegt. Über eine Düse wird ein Gemisch aus Polylactid und Methylcellulose im Verhältnis 1 : 10, welches in Chloroform gelöst vorliegt, und welches auf eine Temperatur von 60° C erwärmt wurde, im Wirbelschichtverfahren auf die Festkörperpartikel aufgebracht.

Es werden Mikrokapseln mit einem Gehalt an einem in fester Form vorliegenden Gemisch gemäß Beispiel 1 in hoher Ausbeute erhalten.

### Beispiel 14

| Aufbaucrème für die Nacht | |
|---|---|
| | Gew.-% |
| Glyceryl-Sorbitan-Oleostearat | 5,00 |
| Ceresin | 3,00 |
| Miglyol 812 | 3,60 |
| Capryl/Caproyl/Isostearyl/Adipin-Triglycerid | 1,00 |
| Isocetylstearat | 6,00 |
| PEG-22-Dodecyl-Glycol-Copolymer | 1,50 |
| Olivenöl | 2,00 |
| Mikrokapseln gemäß Beispiel 11 | 5,00 |
| MgSO₄ | 0,60 |
| Polyhexamethylenbiguanid | 0,20 |
| Tocopherylacetat | 0,50 |
| Cyclomethicon | 2,00 |
| Kollan PM | 0,50 |
| Fibronectin | 0,50 |
| Parfum, Farb- und Konservierungsstoffe | q.s. |
| H₂O | ad 100,00 |

### Beispiel 15

| Tagesintensivcrème | |
|---|---|
| | Gew.-% |
| Glyceryl-Sorbitan-Oleostearat | 5,00 |
| Ceresin | 3,00 |
| Miglyol 812 | 4,10 |
| Capryl/Caproyl/Isostearyl/Adipin-Triglycerid | 1,00 |
| Isocetylstearat | 6,00 |
| PEG-45-Dodecyl-Glycol-Copolymer | 1,50 |
| Octylmethoxycinnamat | 1,00 |
| Butyl-methoxydibenzoylmethan | 0,50 |
| Mikrokapseln gemäß Beispiel 12 | 6,20 |
| MgSO₄ | 0,60 |
| EDTA-Lösung | 0,50 |
| Isohexadecan | 2,00 |
| Tocopherylacetat | 0,50 |
| Kollan PM | 0,50 |
| Fibronectin | 0,50 |
| Parfum, Farb- und Konservierungsstoffe | q.s. |
| H₂O | ad 100,00 |

### Beispiel 16

| Körperlotion | |
|---|---|
| | Gew.-% |
| Arlatone 985 | 4,00 |
| Brij 78 | 2,00 |
| Miglyol 812 | 5,00 |
| Paraffinöl DAB 9 | 5,00 |
| Mikrokapseln gemäß Beispiel 13 | 5,00 |
| Parfum, Farb- und Konservierungsstoffe | q.s. |
| H₂O | ad 100,00 |

### Beispiel 17

| Reinigungsemulsion | |
|---|---|
| | Gew.-% |
| Cutina MD | 4,00 |
| Lanette O | 2,00 |
| Emulgin B1 | 1,50 |
| Rilanit GMO | 1,50 |
| Cetiol SN | 1,00 |
| Paraffin subl. DAB 8 | 10,00 |
| Mikrokapseln gemäß Beispiel 11 | 25,00 |
| Propylenglycol | 5,00 |
| Paraffinöl DAB 9 | 0,50 |
| Parfum, Farb- und Konservierungsstoffe | q.s. |
| H₂O | ad 100,00 |

### Beispiel 18

| Lipo-Crème | |
|---|---|
| | Gew.-% |
| Bienenwachs und PEG-8 | 8,00 |
| Cetylalkohol | 1,00 |
| C₁₂-C₁₅-Alkohol-Benzoat | 7,00 |
| Octyldodecylmyristat | 5,00 |
| Cyclomethicone | 2,00 |
| Tocopherylacetat | 1,10 |
| NaOH | 0,14 |
| Carbomer 934 | 0,35 |
| Mikrokapseln gemäß Beispiel 12 | 5,00 |
| Alkohol SD 39-C | 1,00 |
| Parfum, Farb- und Konservierungsstoffe | q.s. |
| H₂O | ad 100,00 |

### Beispiel 19

| W/O-Emulsion, flüssig | |
|---|---|
| | Gew.-% |
| Elfacos E 200 | 2,00 |
| Elfacos ST 9 | 5,00 |
| Elfacos C 26 | 5,00 |
| Isopropylstearat | 20,00 |
| Mikrokapseln gemäß Beispiel 13 | 5,00 |
| Parfum, Farb- und Konservierungsstoffe | q.s. |
| H₂O | ad 100,00 |

### Beispiel 20

| After Sun Lotion | |
|---|---|
| | Gew.-% |
| Solulan PB 20 | 10,00 |
| Solulan PB 2 | 5,00 |
| Isopropylpalmitat | 10,00 |
| Carbopol 934 P | 0,75 |
| NaOH | 2,25 |
| Ethomeen C25 (10 %-ig) | 3,75 |
| Mikrokapseln gemäß Beispiel 11 | 5,00 |
| Parfum, Farb- und Konservierungsstoffe | q.s. |
| H₂O | ad 100,00 |

### Beispiel 21

| Body Splash | |
|---|---|
| | Gew.-% |
| Ethanol | 40,00 |
| Carbopol 940 | 0,40 |
| Diisopropanolamin | 0,40 |
| Uvinul D50 | 0,05 |
| Polyethylenglycol-2000-monooleat | 5,00 |
| Mikrokapseln gemäß Beispiel 12 | 10,00 |
| Parfum, Farb- und Konservierungsstoffe | q.s. |
| H₂O | ad 100,00 |

### Beispiel 22

| Gesichtswasser | |
|---|---|
| | Gew.-% |
| Chlorhexidingluconat | 0,10 |
| Ethanol | 5,00 |
| Elastin | 3,00 |
| Cremophor RH 40 | 0,20 |
| Mikrokapseln gemäß Beispiel 13 | 4,00 |
| Parfum, Farb- und Konservierungsstoffe | q.s. |
| H₂O | ad 100,00 |

### Beispiel 23

| After Shave | |
|---|---|
| | Gew.-% |
| Tagat S | 2,20 |
| Tegin M | 1,60 |
| Isopropylmyristat | 8,60 |
| Paraffinöl DAB 9 | 8,20 |
| Mikrokapseln gemäß Beispiel 11 | 5,00 |
| Citronensäure | 0,20 |
| KAl(SO₄)₂ * 12 H₂O | 0,10 |
| Milchsäure | 2,00 |
| Bisabolol | 0,07 |
| Parfum, Farb- und Konservierungsstoffe | q.s. |
| H₂O | ad 100,00 |

### Beispiel 24

| Hautgel | |
|---|---|
| | Gew.-% |
| Emulgin B3 | 13,00 |
| Cetiol HE | 20,00 |
| Eutanol G | 5,00 |
| Mikrokapseln gemäß Beispiel 12 | 5,00 |
| Parfum, Farb- und Konservierungsstoffe | q.s. |
| H₂O | ad 100,00 |

### Beispiel 25

| Deospray | |
|---|---|
| | Gew.-% |
| Irgasan DP 300 | 0,10 |
| n-Octyldodecanol | 0,50 |
| Mikrokapseln gemäß Beispiel 11 | 5,00 |
| Ethanol | 33,40 |
| Propan/Butan 2.7 (Treibgas) | ad 100,00 |

### Beispiel 26

| Deo-Stift | |
|---|---|
| | Gew.-% |
| Irgasan DP 300 | 0,20 |
| Natriumstearat | 7,00 |
| 1,2-Propylenglycol | 30,30 |
| Mikrokapseln gemäß Beispiel 11 | 20,00 |
| Triton X 100 | 4,00 |
| Ethanol | 31,00 |
| H₂O VES | 6,00 |

### Beispiel 27

| Desodorierender Pumpspray | |
|---|---|
| | Gew.-% |
| Irgasan DP 300 | 0,20 |
| Triton X 100 | 0,50 |
| Ethanol | 65,00 |
| Mikrokapseln gemäß Beispiel 13 | 12,00 |
| Parfum, Farbstoffe | q.s. |
| H₂O | ad 100,00 |

### Beispiel 28

| Desodorierendes Gel | |
|---|---|
| | Gew.-% |
| Chlorhexidindiacetat | 0,10 |
| Triton X 100 | 3,00 |
| Hydroxyethylcellulose | 0,50 |
| Ethanol | 40,00 |
| Mikrokapseln gemäß Beispiel 13 | 20,00 |
| Parfum, Farbstoffe | q.s. |
| H₂O | ad 100,00 |

### Beispiel 29

| Desodorierende Emulsion | |
|---|---|
| | Gew.-% |
| Irgasan DP 300 | 0,10 |
| Glycerylstearat | 4,00 |
| PEG-40-Cetylstearylalkohol | 3,00 |
| 2-Octyldodecanol | 5,00 |
| Ethanol | 10,00 |
| Polyacrylat-/Methycrylat Copolymer (Lubragel, Sederma) | 0,70 |
| Glycerin | 2,00 |
| Mikrokapseln gemäß Beispiel 13 | 15,00 |
| Parfum, Farbstoffe | q.s. |
| H₂O | ad 100,00 |

## Patentansprüche

1. Verwendung kosmetisch oder pharmazeutisch unbedenklicher Substanzen mit positiver Lösungsenthalpie zur Herstellung Kühlender kosmetischer oder medizinisch topischer Zubereitungen zur Pflege der Haut, wobei die Substanz oder die Substanzen in den Zubereitungen in einem weitgehend wasserfreien Medium vorliegen und/oder durch eine stoffliche Barriere von einem wasserhaltigen Medium abgeschirmt werden.

2. Verwendung nach Anspruch 1, dadurch gekennzeichnet, daß als Substanz oder Substanzen mit positiver Lösungsenthalpie einern oder mehrere Zuckeralkohole der allgemeinen Formel 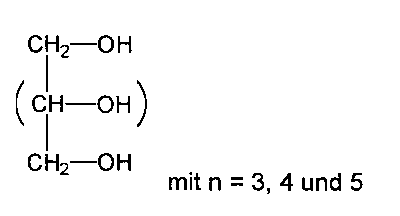 gewählt werden.

3. Verwendung nach Anspruch 2, dadurch gekennzeichnet, daß der Zuckeralkohol Xylitol ist.

4. Verwendung nach Anspruch 1, dadurch gekennzeichnet, daß die stoffliche Barriere darin besteht, daß die Substanz oder die Substanzen mit positiver Lösungsenthalpie mikroverkapselt vorliegen.

5. Verwendung nach Anspruch 3, dadurch gekennzeichnet, daß Xylitol in Kombination mit mindestens einem der Stoffe gewählt aus der Gruppe Mannitol, Sorbitol, Propylenglycol, Glycerin, Harnstoff, 2-Pyrrolidon-5-carbonsäure und deren Natriumsalz sowie Natriummannuronat vorliegt.

6. Verwendung nach Anspruch 5, dadurch gekennzeichnet, daß Kombinationen vorliegen aus:
0,5 - 10,0 Teilen Xylitol
0,0 - 5,0 Teilen Mannitol
0,0 - 5,0 Teilen Sorbitol
0,0 - 5,0 Teilen Glycerin
0,0 - 5,0 Teilen Propylenglycol
0,0 - 5,0 Teilen Harnstoff
0,0 - 5,0 Teilen 2-Pyrrolidon-5-carbonsäure
0,0 - 5,0 Teilen Natrium-(2-Pyrrolidon-5-)carboxylat
0,0 - 5,0 Teilen Natriummannuronat,
bezogen auf die Gesamtzusammensetzung der Zubereitung, mit der Maßgabe, daß mindestens einer der Nicht-Xylitol-Bestandteile in einer Konzentration von wenigstens 0,1 Gew.-% vorliegt, enthalten.

7. Verwendung nach Anspruch 6, dadurch gekennzeichnet, daß Kombinationen vorliegen aus:
0,5 - 10,0 Teilen Xylitol
0,0 - 5,0 Teilen Mannitol
0,0 - 5,0 Teilen Sorbitol
0,0 - 5,0 Teilen Glycerin
0,0 - 5,0 Teilen Propylenglycol
0,0 - 5,0 Teilen Harnstoff
0,0 - 5,0 Teilen 2-Pyrrolidon-5-carbonsäure
0,0 - 5,0 Teilen Natrium-(2-Pyrrolidon-5-)carboxylat
0,0 - 5,0 Teilen Natriummannuronat,
bezogen auf die Gesamtzusammensetzung der Zubereitung, mit der Maßgabe, daß mindestens einer der Nicht-Xylitol-Bestandteile in einer Konzentration von wenigstens 1,0 Gew.-% vorliegt, enthalten.

## Claims

1. Use of cosmetically or pharmacologically acceptable substances having a positive solution enthalpy for the preparation of cooling cosmetic or medicinal topical skin care formulations, the substance, or the substances, being present in the formulations in an essentially anhydrous medium and/or being protected from an aqueous medium by means of a physical barrier.

2. Use according to Claim 1, characterized in that one or more sugar alcohols of the general formula 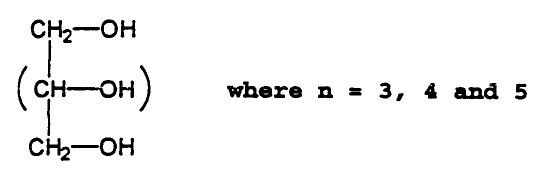 are selected as the substance, or substances, having a positive solution enthalpy.

3. Use according to Claim 2, characterized in that the sugar alcohol is xylitol.

4. Use according to Claim 1, characterized in that the physical barrier consists in the fact that the substance, or the substances, which have a positive solution enthalpy are in microencapsulated form.

5. Use according to Claim 3, characterized in that xylitol is present in combination with at least one of the substances selected from the group comprising mannitol, sorbitol, propylene glycol, glycerol, urea, 2-pyrrolidone-5-carboxylic acid and its sodium salt, and also sodium mannuronate.

6. Use according to Claim 5, characterized in that combinations are present containing
0.5 - 10.0 parts of xylitol
0.0 - 5.0 parts of mannitol
0.0 - 5.0 parts of sorbitol
0.0 - 5.0 parts of glycerol
0.0 - 5.0 parts of propylene glycol
0.0 - 5.0 parts of urea
0.0 - 5.0 parts of 2-pyrrolidone-5-carboxylic acid
0.0 - 5.0 parts of sodium 2-pyrrolidone-5-carboxylate
0.0 - 5.0 parts of sodium mannuronate,
based on the total composition of the formulation, with the proviso that at least one of the non-xylitol components is present at a concentration of at least 0.1 % by weight.

7. Use according to Claim 6, characterized in that combinations are present containing
0.5 - 10.0 parts of xylitol
0.0 - 5.0 parts of mannitol
0.0 - 5.0 parts of sorbitol
0.0 - 5.0 parts of glycerol
0.0 - 5.0 parts of propylene glycol
0.0 - 5.0 parts of urea
0.0 - 5.0 parts of 2-pyrrolidone-5-carboxylic acid
0.0 - 5.0 parts of sodium 2-pyrrolidone-5-carboxylate
0.0 - 5.0 parts of sodium mannuronate,
based on the total composition of the formulation, with the proviso that at least one of the non-xylitol components is present at a concentration of at least 1.0 % by weight.

## Revendications

1. Utilisation de substances cosmétiquement ou pharmaceutiquement acceptables, à enthalpie positive de dissolution, pour la production de préparations topiques cosmétiques ou médicales rafraîchissantes pour le soin de la peau, la substance ou les substances dans les préparations étant présente(s) dans un milieu essentiellement anhydre et/ou étant protégée(s) d'un milieu aqueux par une barrière matérielle.

2. Utilisation selon la revendication 1, caractérisée en ce que l'on choisit comme substance ou substances à enthalpie positive de dissolution un ou plusieurs alcools glucidiques de formule générale

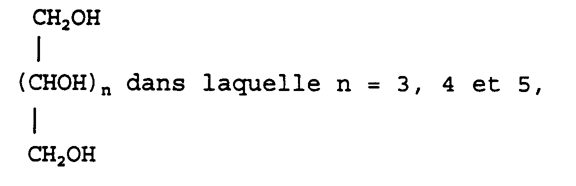

3. Utilisation selon la revendication 2, caractérisée en ce que l'alcool glucidique est le xylitol.

4. Utilisation selon la revendication 1, caractérisée en ce que la barrière matérielle consiste en ce que la substance ou les substances à enthalpie positive de dissolution sont microencapsulées.

5. Utilisation selon la revendication 3, caractérisée en ce que le xylitol est présent en association avec au moins l'une des substances choisies dans l'ensemble constitué par le mannitol, le sorbitol, le propylèneglycol, le glycérol, l'urée, l'acide 2-pyrrolidone-5-carboxylique et son sel de sodium, ainsi que le mannuronate de sodium.

6. Utilisation selon la revendication 5, caractérisée en ce que sont contenues des associations de:
0,5 - 10,0 parties de xylitol
0,0 - 5,0 parties de mannitol
0,0 - 5,0 parties de sorbitol
0,0 - 5,0 parties de glycérol
0,0 - 5,0 parties de propylèneglycol
0,0 - 5,0 parties d'urée
0,0 - 5,0 parties de (2-pyrrolidone-5-)carboxylate de sodium
0,0 - 5,0 parties de mannuronate de sodium
par rapport à la composition totale de la préparation, à la condition qu'au moins l'un des composants à l'exception du xylitol se trouve à une concentration d'au moins 0,1% en poids.

7. Utilisation selon la revendication 6, caractérisée en ce que sont contenues des associations de:
0,5 - 10,0 parties de xylitol
0,0 - 5,0 parties de mannitol
0,0 - 5,0 parties de sorbitol
0,0 - 5,0 parties de glycérol
0,0 - 5,0 parties de propylèneglycol
0,0 - 5,0 parties d'urée
0,0 - 5,0 parties d'acide 2-pyrrolidone-5-carboxylique
0,0 - 5,0 parties de (2-pyrrolidone-5-)carboxylate de sodium
0,0 - 5,0 parties de mannuronate de sodium
par rapport à la composition totale de la préparation, à la condition qu'au moins l'un des composants à l'exception du xylitol se trouve à une concentration d'au moins 1,0% en poids.
